# EUROPÄISCHE PATENTSCHRIFT

(11) **EP 3 069 680 B1**
(45) Veröffentlichungstag und Bekanntmachung des Hinweises auf die Patenterteilung: **03.04.2019**
(21) Anmeldenummer: 16164623.7
(22) Anmeldetag: 25.10.2012
(51) Int. Cl.: A61C 1/00, A61B 17/16, A61B 17/00

(54) **VERFAHREN ZUR STEUERUNG EINER MIT DRUCKGAS BETREIBBAREN MEDIZINISCHEN ANTRIEBSVORRICHTUNG UND DERARTIGE ANTRIEBSVORRICHTUNG**
METHOD FOR CONTROLLING THE DRIVE OF A MEDICAL DRIVE OPERATED BY COMPRESSED-GAS AND SUCH A DRIVE DEVICE
PROCÉDÉ DE CONTRÔLE D'UN INSTRUMENT MÉDICAL A ENTRAINEMENT À GAZ SOUS PRESSION ET DISPOSITIF D'ENTRAINEMENT CORRESPONDANT

(43) Veröffentlichungstag der Anmeldung: 21.09.2016
(62) Teilanmeldung aus: 12189921.5
(73) Patentinhaber: W & H Dentalwerk Bürmoos GmbH, 5111 Bürmoos (AT)
(72) Erfinder: ROTHENWÄNDER, Michael, 5114 Göming (AT); EDER, Karlheinz, 5152 Michaelbeuern (AT); PRUCKNER, Christian, 1190 Wien (AT)
(74) Vertreter: Benda, Ralf

(56) Entgegenhaltungen:
- EP-A1- 1 733 694
- EP-A1- 2 449 999
- DE-A1- 2 839 632
- US-A- 4 723 911
- US-A1- 2008 145 817

## Beschreibung

Die vorliegende Erfindung betrifft Verfahren zur Steuerung oder Regelung einer mit Druckgas betreibbaren medizinischen, insbesondere dentalen, Antriebsvorrichtung und eine derartige Antriebsvorrichtung.

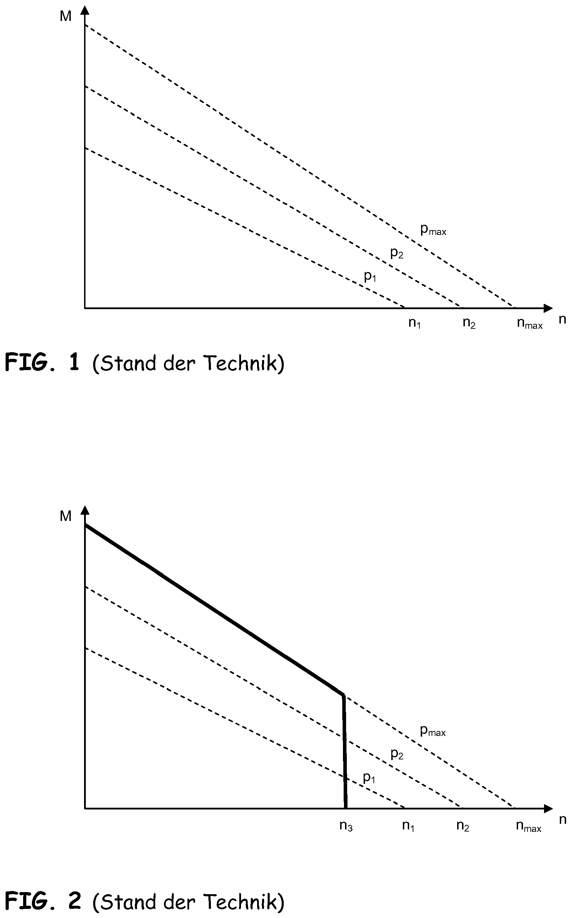

Aus dem Stand der Technik bekannte Verfahren zur Steuerung oder Regelung einer mit Druckgas betreibbaren medizinischen, insbesondere dentalen, Antriebsvorrichtung sind in den Figuren 1 und 2 anhand von Drehmoment-Drehzahl-Diagrammen schematisch dargestellt. Beide Figuren zeigen jeweils ein Drehmoment-Drehzahl-Diagramm einer in Drehung versetzbaren Antriebsvorrichtung, insbesondere eines mit Druckgas beaufschlagbaren Laufrads und / oder einer damit verbundenen Werkzeughaltevorrichtung. Die Drehzahl (n) der Antriebsvorrichtung ist auf der Abszisse aufgetragen, das Drehmoment (M) auf der Ordinate. Des Weiteren sind in den Diagrammen beispielhaft einige wenige Druckverläufe (Geraden p₁, p₂, pₘₐₓ) zu erkennen, die jeweils einen Gasdruckwert des Druckgases wiedergeben. Der Druckwert pₘₐₓ ist der höchste zur Verfügung stehende Druckgaswert, wobei üblicherweise die Antriebsvorrichtung ein Ventil aufweist, welches den Gasdruck auf den Höchstwert pₘₐₓ begrenzt. Jedem Gasdruckwert p₁, p₂, pₘₐₓ entspricht eine Drehzahl n₁, n₂, nₘₐₓ, wobei nₘₐₓ für die höchste, erzielbare Drehzahl steht.

Die Figur 1 zeigt ein Drehmoment-Drehzahl-Diagramm eines bekannten Verfahrens zur Steuerung einer mit Druckgas betreibbaren medizinischen, insbesondere dentalen, Antriebsvorrichtung: Die Antriebsvorrichtung weist ein Steuerventil auf, das von einem Anwender bedienbar ist, zum Beispiel über eine mit dem Fuß bedienbare Stellvorrichtung (im Folgenden als Fußsteuerung bezeichnet), und das ausgebildet ist, den Gasdruck und / oder den Durchfluss des Druckgases entsprechend der Stellung der Fußsteuerung zu steuern und damit die Drehzahl des Laufrads festzulegen. Wählt der Anwender im Leerlauf, zum Beispiel durch Betätigen der Fußsteuerung und damit des Steuerventils, den Druckwert p₁ als den gewünschten Druck aus, so rotiert das Laufrad, mit der Drehzahl n₁, entsprechendes gilt für jeden weiteren wählbaren Druck und die zugehörige Drehzahl, siehe zum Beispiel den Druckwert p₂ und die Drehzahl n₂ sowie den maximalen Druckwert pₘₐₓ und die maximale Drehzahl nₘₐₓ. Entsprechend ist das Drehmoment der Antriebsvorrichtung oder des Laufrads im Leerlauf zumindest nahe null. Im Leerlauf bedeutet, dass ein in der Werkzeughaltevorrichtung gehaltenes Werkzeug ein zu bearbeitendes Teil, insbesondere ein Körpergewebe wie einen Zahn, einen Knochen oder ein Ersatzmaterial, nicht kontaktiert, und somit nicht belastet sind.

Kontaktiert der Anwender mit dem Werkzeug ein zu bearbeitendes Teil oder belastet der Anwender das Werkzeug, dann nimmt, je nach Stärke der Belastung, die Drehzahl gemäß dem Verlauf der dem jeweiligen Druckwert p₁, p₂, pₘₐₓ zugeordneten Geraden ab und das Drehmoment nimmt entsprechend zu. Ist die Drehzahl gleich null, dann erreicht das Drehmoment seinen Höchstwert.

Das Bearbeiten eines Körpergewebes umfasst somit ein beständiges direktes bzw. indirektes Ändern der Drehzahl, des Drehmoments und des Gasdrucks durch den Anwender, zum Beispiel über die Fußsteuerung, ausschließlich auf Basis seiner Wahrnehmung, zum Beispiel der visuell beobachteten Abtragleistung oder Geschwindigkeit der Drehung des Werkzeugs, der mit der Hand auf das zu bearbeitende Teil aufzubringenden Kraft oder der Frequenz der Antriebsvorrichtung. Unterschiedliche Einstellungen der Drehzahl, des Drehmoments und / oder des Gasdrucks, wie sie zum Beispiel für unterschiedliche Behandlungen gewünscht sind, kann der Anwender daher nur näherungsweise auf Basis seiner Wahrnehmung erzielen.

Die Figur 2 zeigt ein Drehmoment-Drehzahl-Diagramm eines zweiten bekannten Verfahrens zur Regelung der Drehzahl einer mit Druckgas betreibbaren medizinischen, insbesondere dentalen, Antriebsvorrichtung: Das Verfahren der Figur 2 entspricht dem Verfahren der Figur 1, jedoch ist eine Regelung vorgesehen, wenn das Laufrad einen vorbestimmten Drehzahlwert n₃ erreicht. Der Drehzahlwert n₃ definiert einen DrehzahlGrenzwert, der nicht überschritten wird bzw. auf den durch Regelung der Druckgaszufuhr durch ein Regelventil geregelt wird. Bei einer Steigerung des Gasdrucks oder Durchflusses des Druckgases durch den Anwender wird das Drehmoment des Laufrads oder des damit angetriebenen Werkzeugs erhöht, gegebenenfalls bis der maximale Gasdruck pₘₐₓ erreicht wird. Nimmt die Belastung des Werkzeugs weiter zu, so steigt zwar das Drehmoment noch weiter an, jedoch nimmt die Drehzahl dann entsprechend ab (siehe Verlauf der in Figur 2 hervorgehobenen Drehzahl-Drehmoment-Kennlinie). Ein derartiges Verfahren ist zum Beispiel aus der Patentanmeldung US 2008/0145817 A1 bekannt. Damit steht dem Anwender während des Betriebs für einen Drehzahlwert (n₃) ein definierter Verlauf oder ein vorbestimmtes Verhältnis von Drehzahl, Drehmoment und Gasdruck zur Verfügung, für alle Drehzahlen unterhalb dieses vorbestimmten Drehzahlwerts (n₃) muss sich der Anwender jedoch wieder auf seine Wahrnehmung verlassen, wie dies im Vorstehenden für die Figur 1 beschrieben ist.

Die Patentanmeldung EP 1 733 694 A1 offenbart ein Handstück mit einem mechanischen Element zum Einstellen des Drucks des der Antriebsvorrichtung zugeführten Luftstroms.

Die Offenlegungsschrift DE 28 39 632 A1 beschreibt einen zahnärztlichen Druckluftmotor, in dem ein durch Druckluft in Drehung versetzbarer Rotor angeordnet ist, der eine mit einem Werkzeug verbindbare Rotorwelle aufweist, wobei der Druckluftzufuhrleitung eine Ventileinrichtung zugeordnet ist, die in Abhängigkeit des am Werkzeug erforderlichen Drehmoments die Druckluftzufuhr verändert.

Der vorliegenden Erfindung liegt daher die Aufgabe zugrunde, ein Verfahren zur Steuerung oder Regelung einer mit Druckgas betreibbaren medizinischen, insbesondere dentalen, Antriebsvorrichtung, eine entsprechende Steuer- oder Regelvorrichtung und eine entsprechende Antriebsvorrichtung zu schaffen, welche(s) die im Vorstehenden genannten Nachteile nicht aufweist. Insbesondere sollen das Verfahren, die Steuer- oder Regelvorrichtung und die Antriebsvorrichtung derart ausgebildet sein, dass sie dem Anwender mehr vorgegebene und / oder frei wählbare Behandlungsoptionen bieten, so dass dem Anwender, vorzugsweise für unterschiedliche Behandlungen, jeweils darauf angepasste, exakt definierte und insbesondere auch tatsächlich an der Antriebsvorrichtung eingestellte Betriebsbedingungen zur Verfügung stehen.

Diese Aufgabe wird durch ein Verfahren zur Steuerung oder Regelung einer mit Druckgas betreibbaren medizinischen, insbesondere dentalen, Antriebsvorrichtung gemäß Anspruch 1 und durch eine Steuer- oder Regelvorrichtung gemäß Anspruch 8 gelöst.

Erfindungsgemäß ist ein Verfahren zur Steuerung oder Regelung einer mit Druckgas betreibbaren medizinischen, insbesondere dentalen, Antriebsvorrichtung vorgesehen, bei dem bei Erreichen eines Drehzahlgrenzwertes des Laufrads durch Stellen des Ventils der Gasdruck des Druckgases und die Drehzahl des Laufrads verändert werden.

Im Unterschied zum Stand der Technik werden somit zwei Parameter, nämlich der Gasdruck des Druckgases und die Drehzahl des Laufrads, (gleichzeitig) verändert.

Bei dem im Vorstehenden genannten Verfahren ist vorzugsweise der Gasdruckwert des Druckgases und / oder der Wert der im Wesentlichen konstanten Drehzahl und / oder der Drehzahlgrenzwert durch einen Anwender einstellbar. Dazu ist eine Bedienvorrichtung für den Anwender vorgesehen, die mit der Antriebsvorrichtung verbunden ist oder als Teil der Antriebsvorrichtung ausgebildet ist. Die Bedienvorrichtung kann zum Beispiel zur Auswahl voreingestellter Werte und / oder zur stufenlosen Auswahl, zumindest innerhalb eines begrenzten Bereichs, ausgebildet sein. Zum Einstellen des ausgewählten Gasdruckwerts des Druckgases und / oder des ausgewählten, im Wesentlichen konstanten Drehzahlwerts und / oder des Drehzahlgrenzwerts ist die Bedienvorrichtung zum Beispiel mit einer Steuer- oder Regelvorrichtung verbunden. Die Bedienvorrichtung umfasst zum Beispiel zumindest einen Taster, einen Drehknopf oder ein Touchscreen. Die Bedienvorrichtung ist zum Beispiel per Hand oder Fuß bedienbar.

Bei dem im Vorstehenden beschriebenen Verfahren weist die Antriebsvorrichtung zumindest ein Laufrad, eine mit dem Laufrad verbundene Werkzeughaltevorrichtung und ein Ventil zum Stellen des Gasdrucks und / oder des Durchflusses des Druckgases auf, zum Beispiel ein Steuer- oder Regelventil, vorzugsweise ein Proportionalventil, insbesondere ein Magnetventil. Des Weiteren ist die Antriebsvorrichtung von einer Druckgasquelle mit einem Druckgas mit einem maximalen Gasdruck versorgbar, um das Laufrad in eine Drehbewegung zu versetzen. Vorzugsweise ist die Druckgasquelle als Druckluftquelle ausgebildet, welche die Antriebsvorrichtung mit Druckluft versorgt.

Das im Vorstehenden beschriebene Verfahren bieten dem Anwender in vorteilhafter Weise eine im Vergleich zum Stand der Technik erheblich größere Anzahl von Behandlungsoptionen, vorzugsweise für unterschiedliche Behandlungen. Dem Anwender stehen insbesondere exakt definierte und während der Behandlung auch (kontinuierlich) eingehaltene, gesteuerte oder geregelte oder überwachte Verhältnisse, Verläufe oder Kombinationen von Drehzahlwerten, Drehmomentwerten und Gasdruckwerten zur Verfügung. Des Weiteren hat der Anwender in vorteilhafter Weise die Möglichkeit, exakte Einstellungen der Drehzahl, des Drehmoments und des Gasdrucks und / oder exakte Einstellungen von gewünschten Verhältnissen, Verläufen oder Kombinationen dieser Parameter zu definieren oder auszuwählen.

Vorzugsweise umfasst das Verfahren zur Steuerung oder Regelung einer mit Druckgas betreibbaren medizinischen, insbesondere dentalen, Antriebsvorrichtung eine Drehzahluntergrenze, unterhalb der das Verfahren zur Steuerung oder Regelung nicht durchgeführt wird, wobei die Drehzahluntergrenze > 0 s⁻¹ ist. Die Drehzahluntergrenze ist entweder vom Anwender frei wählbar oder sie ist unveränderbar festgesetzt. Die Drehzahluntergrenze bietet dem Anwender in vorteilhafter Weise eine weitere Möglichkeit zur individuellen Gestaltung von Behandlungen.

Vorzugsweise ist bei dem Verfahren zur Steuerung oder Regelung einer mit Druckgas betreibbaren medizinischen, insbesondere dentalen, Antriebsvorrichtung vorgesehen, wenn die Antriebsvorrichtung oder das Laufrad nicht mit Druckgas versorgt werden oder die Antriebsvorrichtung nicht betrieben wird oder das Laufrad nicht rotiert, zumindest einer der folgenden Parameter durch den Anwender zumindest innerhalb eines vordefinierten Bereichs frei wählbar ist: Ein Drehzahlgrenzwert des Laufrads, der geringer ist als die durch den maximalen Gasdruck pₘₐₓ bestimmte maximale Drehzahl nₘₐₓ, eine Solldrehzahl des Laufrads, ein Gasdruckgrenzwert des (dem Laufrad zuführbaren) Druckgases, der geringer ist als der der Antriebsvorrichtung zur Verfügung stehende maximale Gasdruck (pₘₐₓ), ein Drehmomentwert. Die Antriebsvorrichtung umfasst ein Laufrad, eine mit dem Laufrad verbundene Werkzeughaltevorrichtung und ein Ventil zum Stellen des Gasdrucks und / oder des Durchflusses des Druckgases, wobei die Antriebsvorrichtung von einer Druckgasquelle mit dem Druckgas mit einem maximalen Gasdruck versorgbar ist.

Die Auswahl eines Wertes zumindest eines dieser Parameter ermöglicht dem Anwender in vorteilhafter Weise eine individuelle Gestaltung von Behandlungen.

Zur Auswahl eines Wertes zumindest eines dieser Parameter ist eine Bedienvorrichtung für den Anwender vorgesehen, die mit der Antriebsvorrichtung verbunden ist oder als Teil der Antriebsvorrichtung ausgebildet ist. Die Bedienvorrichtung kann zum Beispiel zur Auswahl voreingestellter Werte und / oder zur stufenlosen Auswahl, zumindest innerhalb eines begrenzten Bereichs, ausgebildet sein. Zum Einstellen eines Wertes ist die Bedienvorrichtung zum Beispiel mit einer Steuer- oder Regelvorrichtung und / oder mit einem Stellelement, insbesondere dem Ventil, verbunden. Das Stellelement ist ausgebildet, eine Änderung der Drehzahl des Laufrads, der Solldrehzahl des Laufrads, des maximalen Gasdrucks des Druckgases oder des Drehmoments zu bewirken, insbesondere durch Einwirken auf das Druckgas, zum Beispiel durch Veränderung des Querschnitts einer Ventilöffnung, durch welche das Druckgas strömt. Die Bedienvorrichtung umfasst zum Beispiel zumindest einen Taster, einen Drehknopf oder ein Touchscreen. Die Bedienvorrichtung ist zum Beispiel per Hand oder Fuß bedienbar.

Gemäß einem Ausführungsbeispiel ist ein Verfahren zur Erkennung von Verschleiß oder eines Defekts zumindest eines Teils einer Antriebsvorrichtung vorgesehen, wobei bei dem Verfahren ein Gasdruckwert des Druckgases zum Antrieb der Antriebsvorrichtung, insbesondere des Laufrads, vorgegeben ist oder wird, die Antriebsvorrichtung, insbesondere das Laufrad, durch Beaufschlagen mit Druckgas mit dem vorgegebenen Gasdruckwert in Drehung versetzt wird, die Drehzahl der Antriebsvorrichtung, insbesondere des Laufrads, von einer Drehzahlmessvorrichtung bestimmt und an eine Steuer- oder Regelvorrichtung übertragen wird, der gemessene Drehzahlwert durch die Steuer- oder Regelvorrichtung mit einem Drehzahl-Sollwert verglichen wird, wobei der Drehzahl-Sollwert durch eine dem vorgegebenen Gasdruckwert zugeordnete Drehzahl gebildet ist, und wenn der gemessene Drehzahlwert ungleich dem Drehzahl-Sollwert ist oder sich außerhalb eines den Drehzahl-Sollwert umfassenden Toleranzbereichs befindet, der Betrieb der Antriebsvorrichtung verhindert wird und / oder eine Anzeige zur Information des Anwenders aktiv wird.

Vorzugsweise wird das Verfahren zur Erkennung von Verschleiß oder eines Defekts im Leerlauf oder ohne Belastung der Antriebsvorrichtung, insbesondere ohne Belastung eines durch die Antriebsvorrichtung angetriebenen Werkzeugs, oder ohne Kontakt der Antriebsvorrichtung oder eines davon angetriebenen Werkzeugs zu einem zu bearbeitenden Objekt durchgeführt. Vorzugsweise ist der Drehzahl-Sollwert durch die dem vorgegebenen Gasdruckwert zugeordnete Leerlauf-Drehzahl gebildet. Zum Beispiel umfasst der den Drehzahl-Sollwert umfassende Toleranzbereich +/- 5% oder +/- 10% des Drehzahl-Sollwerts.

Das Verfahren zur Erkennung von Verschleiß oder eines Defekts ermöglicht insbesondere die Erkennung von verschlissenen oder defekten Lagern, die insbesondere das Laufrad und / oder die Werkzeughaltevorrichtung lagern, oder von Objekten in der Antriebsvorrichtung oder von Bauteilen der Antriebsvorrichtung, die sich gelöst haben oder lose sind und ein rotierendes Bauteil der Antriebsvorrichtung, insbesondere das Laufrad und / oder die Werkzeughaltevorrichtung, kontaktieren.

Der Betrieb der Antriebsvorrichtung wird zum Beispiel durch Unterbindung der Zufuhr des Druckgases an die Antriebsvorrichtung, insbesondere an das Laufrad, unterbunden, zum Beispiel durch vollständigen Verschluss eines Ventils in der Druckgasversorgung der Antriebsvorrichtung. Die Anzeige zur Information des Anwenders umfasst zum Beispiel einen visuellen Signalgeber, zum Beispiel eine Lichtquelle, einen akustischen Signalgeber oder einen taktilen Signalgeber.

Gemäß einem Ausführungsbeispiel ist eine Steuer- oder Regelvorrichtung vorgesehen, die zur Durchführung des im Vorstehenden beschriebenen Verfahrens zur Steuerung oder Regelung einer mit Druckgas betreibbaren medizinischen, insbesondere dentalen, Antriebsvorrichtung und / oder des Verfahrens zur Erkennung von Verschleiß oder eines Defekts zumindest eines Teils einer Antriebsvorrichtung ausgebildet ist. Die Steuer- oder Regelvorrichtung ist elektrisch betrieben und umfasst einen Mikrokontroller. Die Steuer- oder Regelvorrichtung ist vorzugsweise mit der im Vorstehenden beschriebenen Anzeige zur Information des Anwenders operativ verbunden. Die Steuer- oder Regelvorrichtung ist vorzugsweise mit der im Vorstehenden beschriebenen Bedienvorrichtung operativ verbunden. Die Steuer- oder Regelvorrichtung ist vorzugsweise als Teil der medizinischen, insbesondere dentalen, Antriebsvorrichtung ausgebildet oder mit der medizinischen, insbesondere dentalen, Antriebsvorrichtung operativ verbunden.

Die medizinische, insbesondere dentale, Antriebsvorrichtung umfasst neben der im Vorstehenden genannten Steuer- oder Regelvorrichtung ein durch das Druckgas in Bewegung versetzbares Laufrad, eine mit dem Laufrad verbundene Werkzeughaltevorrichtung, eine Drehzahlmessvorrichtung zur Messung der Drehzahl des Laufrads und / oder der Werkzeughaltevorrichtung, ein Ventil zum Stellen des Gasdrucks und / oder des Durchflusses des Druckgases und vorzugsweise eine Vorrichtung zur Bestimmung des Gasdrucks des Druckgases, wobei die Steuer- oder Regelvorrichtung operativ mit der Drehzahlmessvorrichtung, vorzugsweise mit der Vorrichtung zur Bestimmung des Gasdrucks und mit dem Ventil verbunden ist, so dass Drehzahlmessdaten und gegebenenfalls Gasdruckmessdaten an die Steuer- oder Regelvorrichtung übertragbar sind und das Ventil auf Basis der übertragenen Drehzahlmessdaten und / oder gegebenenfalls der Gasdruckmessdaten durch die Steuer- oder Regelvorrichtung zum Stellen des Gasdrucks und / oder des Durchflusses des Druckgases betätigbar ist.

Vorzugsweise umfasst die Antriebsvorrichtung ein Hand- oder Winkelstück, in dem zumindest das Laufrad und die Werkzeughaltevorrichtung vorgesehen sind. Jedes der übrigen genannten Bauteil (die Steuer- oder Regelvorrichtung, die Drehzahlmessvorrichtung, das Ventil, die Vorrichtung zur Bestimmung des Gasdrucks) können im Hand- oder Winkelstück oder außerhalb des Hand- oder Winkelstücks vorgesehen sein, zum Beispiel in einer Kupplungsvorrichtung oder einem Schlauch zur Verbindung des Hand- oder Winkelstücks mit der Druckgasquelle oder in einer Versorgungseinheit. Das Ventil zum Stellen des Gasdrucks und / oder des Durchflusses des Druckgases umfasst zum Beispiel ein Steuer- oder Regelventil, insbesondere ein Magnetventil. Die Drehzahlmessvorrichtung ist zum Beispiel als induktive, kapazitive oder optische Drehzahlmessvorrichtung ausgebildet. Die Vorrichtung zur Bestimmung des Gasdrucks ist zum Beispiel als Mikrofon, als kapazitiver, induktiver oder piezoelektrischer Sensor ausgebildet. Alternativ ist es auch möglich, den Gasdruck indirekt zu bestimmen, vorzugsweise durch Detektion einer Stellgröße des Ventils zum Stellen des Gasdrucks und / oder des Durchflusses des Druckgases, zum Beispiel der Position des Ventilkörpers, oder durch Bestimmen des Wertes eines an dem Ventil anliegenden Steuer- oder Regelsignals, zum Beispiel der Stromstärke.

Gemäß einem Ausführungsbeispiel umfasst die Antriebsvorrichtung zumindest ein von einem Anwender betätigbares Stellelement zum Festlegen zumindest eines der folgenden Parameter: Eines Drehzahlgrenzwerts des Laufrads, der geringer ist als die durch den maximalen Gasdruck bestimmte maximale Drehzahl, einer Solldrehzahl des Laufrads, eines Gasdruckgrenzwerts des Druckgases, der geringer ist als der maximale Gasdruck (pₘₐₓ), welcher der Antriebsvorrichtung zur Verfügung steht, eines Drehmoment(grenz)werts. Das Stellelement ist zum Beispiel mit einer im Vorstehenden beschriebenen Bedienvorrichtung verbunden.

Die Erfindung wird nachfolgend anhand bevorzugter Ausführungsbeispiele und Bezug nehmend auf die beigefügten Zeichnungen erläutert. Es zeigt / zeigen die
Figuren 1 und 2 jeweils ein Drehmoment-Drehzahl-Diagramm von aus dem Stand der Technik bekannten Verfahren zur Steuerung oder Regelung einer mit Druckgas betreibbaren medizinischen, insbesondere dentalen, Antriebsvorrichtung.
Figur 3 und 4 Steuer- oder Regelcharakteristika in Form von Drehmoment-Drehzahl-Diagrammen von Verfahren zur Steuerung und / oder Regelung einer mit Druckgas betreibbaren medizinischen, insbesondere dentalen, Antriebsvorrichtung, wobei die Charakteristika der Figur 3 nicht Gegenstand der Patentansprüche sind.
Figur 5 - 7 unterschiedliche Ausführungsformen von Steuer- oder Regelsettings in Form von Drehmoment-Drehzahl-Diagrammen.
Figur 8 eine medizinische, insbesondere dentale, mit Druckgas betreibbare Antriebsvorrichtung zur Durchführung eines Verfahrens zur Steuerung oder Regelung einer mit Druckgas betreibbaren medizinischen, insbesondere dentalen, Antriebsvorrichtung.

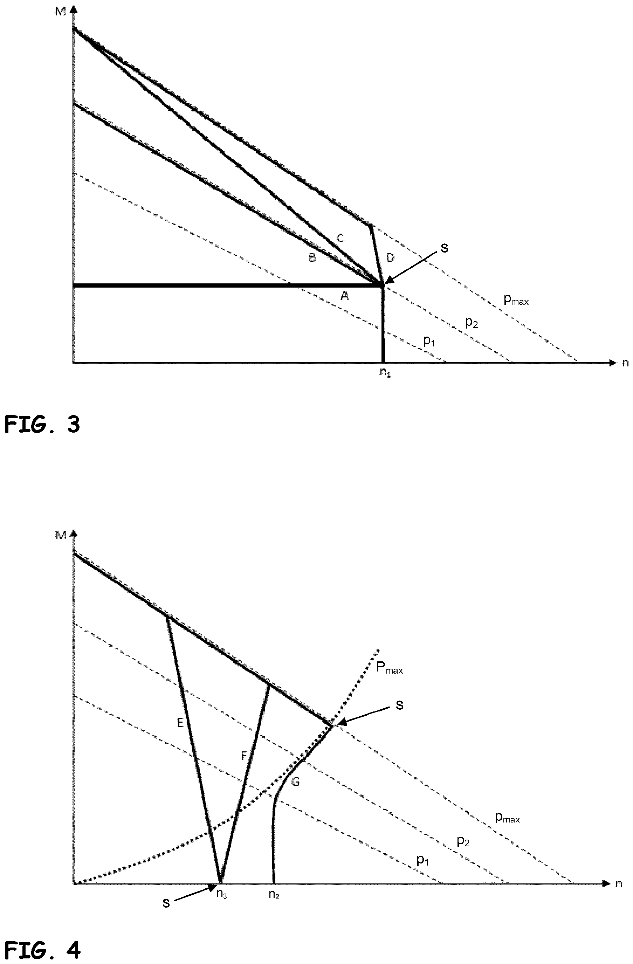

Die Figuren 3 - 7 zeigen Drehmoment-Drehzahl-Diagramme von in Drehung versetzbaren Antriebsvorrichtungen, insbesondere eines mit Druckgas beaufschlagbaren Laufrads und / oder einer damit verbundenen Werkzeughaltevorrichtung. Die Drehzahl (n) der Antriebsvorrichtung ist auf der Abszisse aufgetragen, das Drehmoment (M) auf der Ordinate. Des Weiteren sind in den Diagrammen beispielhaft einige wenige Druckverläufe (Geraden p₁, p₂, pₘₐₓ) zu erkennen, die jeweils einen Gasdruckwert des Druckgases wiedergeben. Der Druckwert pₘₐₓ ist der höchste zur Verfügung stehende Druckgaswert, wobei üblicherweise die Antriebsvorrichtung ein Ventil aufweist, welches den Gasdruck des von der Druckgasquelle zur Verfügung gestellten Druckgases auf den Höchstwert pₘₐₓ begrenzt (pₘₐₓ ist somit der höchste der Antriebsvorrichtung oder dem Laufrad der Antriebsvorrichtung zur Verfügung stehende Gasdruck). Jedem Gasdruckwert p₁, p₂, pₘₐₓ entspricht eine Drehzahl n₁, n₂, nₘₐₓ (nₘₐₓ ist die höchste, erzielbare Drehzahl). Es wird darauf verwiesen, dass alle dargestellten Drehmoment-Drehzahl-Diagramme schematische, näherungsweise Darstellungen zur Erleichterung des Verständnisses sind. Insbesondere sind die als Geraden dargestellten Druckverläufe oder Drehzahl-Gasdruck-Verläufe in der Praxis schwach gebogen (konvex) ausgebildet.

Die Figur 3 zeigt ein Drehmoment-Drehzahl-Diagramm eines ersten Ausführungsbeispiels eines Verfahrens zur Steuerung oder Regelung einer mit Druckgas betreibbaren medizinischen, insbesondere dentalen, Antriebsvorrichtung, bei dem das Laufrad durch Stellen des Gasdrucks und / oder des Durchflusses des Druckgases durch ein Ventil zumindest innerhalb eines begrenzten Belastungsbereichs mit einer im Wesentlichen konstanten Drehzahl n₁ betrieben wird, und bei Erreichen eines Druckwertes p₂ des Druckgases, der geringer ist als der maximale Gasdruck pₘₐₓ des Druckgases, die Drehzahl (gesteuert oder geregelt) reduziert wird. Vorzugsweise ist das Verfahren reversibel, so dass bei Unterschreiten des Druckwertes p₂ die Antriebsvorrichtung oder das Laufrad zum Beispiel wieder mit der im Wesentlichen konstanten Drehzahl n₁ oder mit einem beliebigen anderen Steuer- oder Regelverfahren betrieben werden.

Der Druckwert p₂ oder die Kombination des Druckwertes p₂ und der Drehzahl n₁ definieren somit einen Schaltpunkt S, bei dessen Erreichen die Drehzahl reduziert wird. In Verbindung mit der Reduktion der Drehzahl sind vorzugsweise weitere Änderungen von Werten von Betriebsparametern möglich: Zum Beispiel bleibt mit der Reduktion der Drehzahl das Drehmoment im Wesentlichen konstant oder es erhöht sich nur geringfügig, insbesondere weist das Drehmoment in etwa denselben Wert oder einen nur geringfügig erhöhten Wert wie bei Erreichen des Druckwertes p₂, bei dem die Drehzahl reduziert wird, auf (siehe Figur 3, Gerade "A"). Damit verbunden ist eine Reduktion des Gasdrucks des das Laufrad antreibenden Druckgases. Eine derartige Steuerung oder Regelung ist zum Beispiel bei medizinischen oder dentalen Feinbehandlungen oder Nachbearbeitungen oder bei Behandlungen in Pulpanähe vorteilhaft, insbesondere in Verbindung mit einem niedrigen, im Wesentlichen konstanten Drehzahlwert n₁.

Alternativ nimmt mit der Reduktion der Drehzahl das Drehmoment, insbesondere erheblich, zu. Insbesondere weist das Drehmoment einen, insbesondere erheblich, höheren Wert wie beim Erreichen des Druckwertes p₂, bei dem die Drehzahl reduziert wird, auf. Eine derartige Steuerung oder Regelung ist zum Beispiel bei medizinischen oder dentalen Initialpräparationen oder Bearbeitungen von Zahnschmelz oder von Zahnersatzmaterial vorteilhaft, insbesondere in Verbindung mit einem hohen, im Wesentlichen konstanten Drehzahlwert n₁. Wie aus den Geraden "B", "C" und "D" der Figur 3 zu erkennen ist, gibt es hierbei wiederum unterschiedliche Verläufe: Die Gerade "B" folgt im Wesentlichen dem Druckverlauf p₂, d.h. der Gasdruck des das Laufrad antreibenden Druckgases bleibt im Wesentlichen konstant. Alternativ ist am Verlauf der Geraden "C" zu erkennen, dass der Gasdruck des das Laufrad antreibenden Druckgases zunimmt, insbesondere stetig zunimmt. Die Steigerung des Gasdrucks kann dabei derart ausgebildet sein, dass der Gasdruck sich dem maximalen Druckgaswert pₘₐₓ annähert, diesen jedoch nicht erreicht, oder dass der Gasdruck sich dem maximalen Druckgaswert pₘₐₓ annähert und diesen erst bei sehr geringen Drehzahlen erreicht (siehe Gerade "C" der Figur 3). Eine weitere Alternative ist durch die Gerade "D" der Figur 3 dargestellt, bei welcher der Gasdruck des das Laufrad antreibenden Druckgases stark und insbesondere stetig zunimmt, so dass der Gasdruck den maximalen Druckgaswert pₘₐₓ erreicht und bei weiterer Belastung (zunehmendem Drehmoment) beibehält.

Die als Geraden "A" - "D" dargestellten Verläufe und das Verhältnis der Drehzahl, des Gasdrucks und des Drehmoments zueinander sind selbstverständlich exemplarisch, insbesondere sind die Steigungen der Geraden "B" - "D" beliebig variabel, solange bei Erreichen eines Druckwertes p₂ des Druckgases, der geringer ist als der maximale Gasdruck pₘₐₓ des Druckgases, eine Reduktion der Drehzahl erfolgt. Klarerweise müssen die Verläufe oder das Verhältnis der Drehzahl, des Gasdrucks und des Drehmoments zueinander auch nicht als Gerade ausgebildet sein, sondern können auch durch nicht stetige oder gebogene oder geknickte Verläufe oder durch Kurven definiert sein.

Die Figur 4 zeigt ein Drehmoment-Drehzahl-Diagramm eines zweiten Ausführungsbeispiels eines Verfahrens zur Steuerung oder Regelung einer mit Druckgas betreibbaren medizinischen, insbesondere dentalen, Antriebsvorrichtung, bei dem bei Erreichen eines Drehzahlgrenzwertes n₂, n₃ des Laufrads durch Stellen des Ventils der Gasdruck des Druckgases und (gleichzeitig) die Drehzahl des Laufrads (gesteuert oder geregelt) verändert werden (siehe insbesondere die Verläufe der Geraden "E" und "F" der Figur 4). Vorzugsweise werden der Gasdruck des Druckgases und (gleichzeitig) die Drehzahl des Laufrads verändert, wenn zusätzlich zum Erreichen eines Drehzahlgrenzwertes n₂ auch ein Druckwert p₁ des Druckgases, der geringer ist als der maximale Gasdruck pₘₐₓ des Druckgases, erreicht wird (siehe den Verlauf der Kurve "G" der Figur 4). Insbesondere kann vor Erreichen des Druckwertes p₁ das Laufrad mit einer im Wesentlichen konstanten Drehzahl n₂ betrieben werden. Der Drehzahlgrenzwert n₂, n₃ kann wahlweise im Leerlauf (ohne Belastung, siehe Kurven "E" und "F") oder unter Belastung (bei Anliegen eines Drehmoments, siehe Kurve "G") erreichbar sein. Der Drehzahlgrenzwert n₂, n₃ oder die Kombination des Drehzahlgrenzwertes n₂, n₃ und des Druckwert p₁ definieren somit einen Schaltpunkt S, bei dessen Erreichen der Gasdruck des Druckgases und (gleichzeitig) die Drehzahl des Laufrads verändert werden.

Vorzugsweise ist das Verfahren reversibel, so dass bei Unterschreiten des Drehzahlgrenzwertes n₂, n₃ oder gegebenenfalls des Druckwertes p₁ die Antriebsvorrichtung oder das Laufrad zum Beispiel wieder mit einem beliebigen anderen Steuer- oder Regelverfahren betrieben werden.

Die dargestellten Verläufe der Geraden "E", "F" und der Kurve "G" und das Verhältnis der Drehzahl, des Gasdrucks und des Drehmoments zueinander sind selbstverständlich wiederum exemplarisch, insbesondere sind zum Beispiel die Steigungen beliebig variabel. Beispielhaft sind bei Erreichen eines Drehzahlgrenzwertes n₂, n₃ und gegebenenfalls des Druckwert p₁ folgende Verläufe der Geraden "E", "F" und der Kurve "G" oder Verhältnisse der Drehzahl, des Gasdrucks und des Drehmoments zueinander möglich: Die Drehzahl (des Laufrads) nimmt ab und der Gasdruck des Druckgases und das Drehmoment steigen (siehe Gerade "E"). Dabei ist es möglich, dass der Gasdruck derart steigt, dass der maximale Gasdruck pₘₐₓ erreicht und gegebenenfalls bei weiterer Abnahme der Drehzahl beibehalten wird (siehe Gerade "E") oder dass alternativ der maximale Gasdruck pₘₐₓ nicht erreicht wird. Alternativ, gemäß der Gerade "F" und der Kurve "G" steigen die Drehzahl des Laufrads, der Gasdruck des Druckgases und das Drehmoment. Der Gasdruck des Druckgases steigt maximal bis zum Erreichen des maximalen Gasdrucks pₘₐₓ, wird die Belastung (das Drehmoment) dann weiter erhöht, so nimmt die Drehzahl unter Beibehaltung des maximalen Gasdrucks pₘₐₓ ab. Aus dem Verlauf der Kurve "G" ist des Weiteren erkennbar, dass die Zunahme des Gasdrucks und der Drehzahl vorzugsweise dem Verlauf der maximalen Leistung Pₘₐₓ (entspricht der halben Leerlaufdrehzahl) der Antriebsvorrichtung folgt.

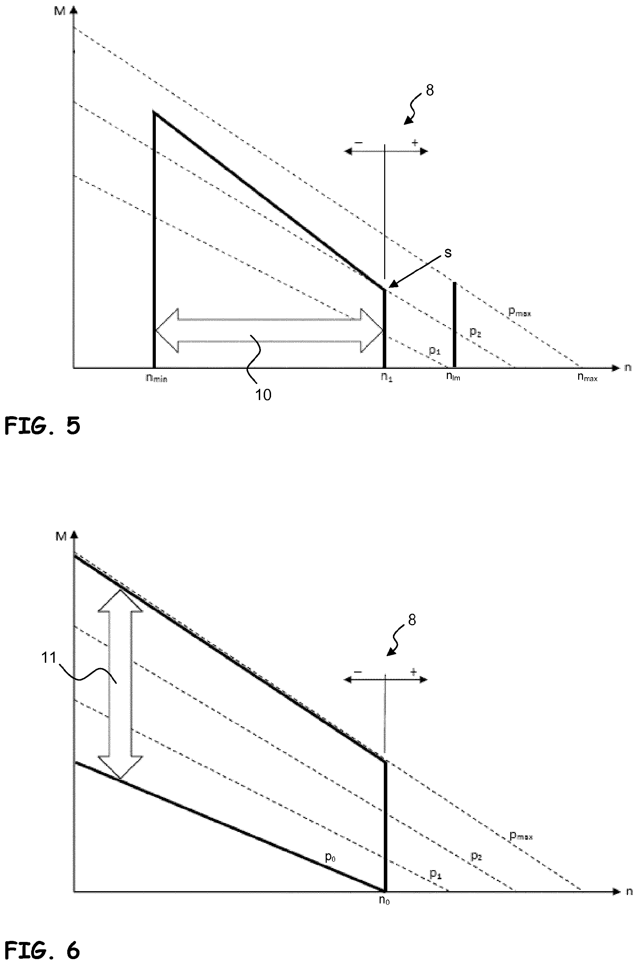

Das in der Figur 5 dargestellt Drehmoment-Drehzahl-Diagramm zeigt ein Beispiel wie eine im Vorstehenden beschriebene Steuer- oder Regelcharakteristik mit einem Steuer- oder Regelsetting verbindbar ist: Es sind eine als Solldrehzahl definierte Drehzahl n₁, vorzugsweise eine minimale Drehzahl oder Drehzahluntergrenze nₘᵢₙ und vorzugsweise eine Drehzahlobergrenze nₗₘ vorgesehen. Die Drehzahlobergrenze nₗₘ ist dabei von der maximalen Drehzahl nₘₐₓ zu unterscheiden, die durch den maximalen, der Antriebsvorrichtung zur Verfügung stehenden Gasdruck pₘₐₓ festgelegt ist. Der Wert der Drehzahlobergrenze nₗₘ ist bevorzugt kleiner als der Wert der maximalen Drehzahl nₘₐₓ und begrenzt die Drehzahl des Laufrads, zum Beispiel zur Schonung der das Laufrad lagernden Wälzlager. Die Drehzahlobergrenze nₗₘ legt insbesondere eine Grenze fest, die von der durch den Anwender einstellbaren Solldrehzahl n₁ nicht überschritten werden kann (d.h. n₁ < nₗₘ oder n₁ <= nₗₘ). Der Wert der Solldrehzahl n₁ ist vorzugsweise mit einem von einem Anwender betätigbaren Stellelement 8 (in der Figur 5 symbolisch dargestellt), zum Beispiel über eine Bedienvorrichtung mit einem Drehknopf oder einem Taster, insbesondere mit einem per Hand betätigbaren Stellelement, einstellbar. Die minimale Drehzahl nₘᵢₙ und die Drehzahlobergrenze nₗₘ sind vorzugsweise für den Anwender nicht veränderbar voreingestellt, es ist jedoch auch denkbar, dass zumindest einer dieser beiden Parameter mit einem von einem Anwender betätigbaren Stellelement, zum Beispiel über eine Bedienvorrichtung mit einem Drehknopf oder einem Taster, veränderbar ist.

Wie durch den Pfeil 10 in der Figur 5 verdeutlicht, kann der Anwender mittels eines Stellelements oder einer Bedienvorrichtung, vorzugsweise mittels eines vom Stellelement 8 unterschiedlichen Stellelement, insbesondere mittels eines mit dem Fuß betätigbaren Stellelement, die Drehzahl während des Betriebs der Antriebsvorrichtung innerhalb der Drehzahlgrenzen nₘᵢₙ und nₗₘ beliebig vorgeben oder einstellen. Bei Erreichen der Solldrehzahl n₁ und Zunahme des Drehmoments bleibt die Drehzahl im Wesentlichen konstant (die Solldrehzahl n₁ wird im Wesentlichen konstant beibehalten oder eine Steuer- oder Regelvorrichtung steuert / regelt auf diese Drehzahl, so dass das Laufrad mit einer im Wesentlichen konstanten Drehzahl betrieben wird), wobei der Gasdruck des Druckgases zunimmt. Bei Erreichen des Schaltpunkts S wird die Drehzahl reduziert, so wie dies im Vorstehenden für die Figur 3, Gerade "C" beschrieben ist. Bei Erreichen der Drehzahluntergrenze nₘᵢₙ wird die Zufuhr des Druckgases zur Antriebsvorrichtung oder zum Laufrad unterbunden.

Selbstverständlich ist es möglich, das in der Figur 5 dargestellte Steuer- oder Regelsetting mit anderen Steuer- oder Regelcharakteristiken zu verbinden, insbesondere mit den in den Figuren 3 und 4 dargestellten Steuer- oder Regelcharakteristiken "A", "B", "D", "E", "F" oder "G" oder beliebigen anderen Steuer- oder Regelcharakteristiken.

Eine Steuer- oder Regelvorrichtung mit einem Mikrokontroller ist vorgesehen, die / der zur Durchführung der Steuer- oder Regelcharakteristik der Figur 5 ausgebildet ist. Die Steuer- oder Regelvorrichtung ist mit den Stellelementen und zumindest einem Sensor, insbesondere einem Drehzahlsensor, operativ verbunden und ist des Weiteren insbesondere dazu ausgebildet, von dem zumindest einen Sensor Signale zu erhalten, mit Vorgabewerten (zum Beispiel der Solldrehzahl n₁, Drehzahlobergrenze nₗₘ oder der Drehzahluntergrenze nₘᵢₙ) zu vergleichen und ein Signal zum Stellen des auf das Druckgas einwirkenden Ventils abzugeben.

Gemäß dem Steuer- oder Regelsetting der Figur 6 kann der Anwender mittels eines Stellelements und / oder einer Bedienvorrichtung, insbesondere mit einem mit dem Fuß betätigbaren Stellelement, das Drehmoment oder den Gasdruck des Druckgases beliebig vorgeben oder einstellen. Gemäß diesem auch eigenständigen erfinderischen Aspekt ist somit ein Verfahren zur Steuerung oder Regelung einer mit Druckgas betreibbaren medizinischen, insbesondere dentalen, Antriebsvorrichtung mit einem Laufrad und einer mit dem Laufrad verbundenen Werkzeughaltevorrichtung vorgesehen, wobei die Antriebsvorrichtung von einer Druckgasquelle mit dem Druckgas (mit einem maximalen Gasdruck) versorgbar ist, um das Laufrad in eine Drehbewegung zu versetzen, und wobei die Antriebsvorrichtung ein Ventil zum Stellen des Gasdrucks und / oder des Durchflusses des Druckgases aufweist, wobei das Verfahren dadurch definiert ist, dass als zu steuernde Größe oder als Regelgröße das Drehmoment der Antriebsvorrichtung oder des Laufrads oder der Gasdruck des die Antriebsvorrichtung oder das Laufrad antreibenden Druckgases vorgesehen ist.

Wie durch den Pfeil 11 verdeutlicht, kann der Anwender mittels der Fußsteuerung das Drehmoment oder den Gasdruck während des Betriebs der Antriebsvorrichtung innerhalb von Drehmoment- oder Gasdruckgrenzen beliebig vorgeben oder einstellen. Die Untergrenze ist in der Figur 6 mit p₀ bezeichnet und kann gleich oder größer 0 bar sein. Die Untergrenze p₀ ist entweder für den Anwender unveränderbar vorgegeben oder durch ein durch den Anwender betätigbares Stellelement, insbesondere über eine Bedienvorrichtung mit zum Beispiel einem Taster oder einem Drehknopf, vorzugsweise mittels eines mit der Hand betätigbaren Stellelements, stellbar.

Die Obergrenze ist in der Figur 6 mit pₘₐₓ bezeichnet und gleich dem maximalen, der Antriebsvorrichtung oder dem Laufrad zur Verfügung stehenden Gasdruck. Alternativ kann die Obergrenze geringer sein als der maximale Gasdruck pₘₐₓ. In letzterem Fall ist die Obergrenze entweder für den Anwender unveränderbar vorgegeben oder durch ein durch den Anwender betätigbares Stellelement, insbesondere über eine Bedienvorrichtung mit zum Beispiel einem Taster oder einem Drehknopf, vorzugsweise mittels eines mit der Hand betätigbaren Stellelements, stellbar.

Bevorzugt weist das Steuer- oder Regelsetting der Figur 6 des Weiteren einen Drehzahlgrenzwert n₀ auf. Bei Erreichen des Drehzahlgrenzwerts n₀ und steigender Belastung bleibt die Drehzahl im Wesentlichen konstant, während der Gasdruck ansteigt. Der Drehzahlgrenzwert n₀ ist für den Anwender entweder unveränderbar vorgegeben oder bevorzugt durch ein durch den Anwender betätigbares Stellelement 8, insbesondere über eine Bedienvorrichtung mit zum Beispiel einem Taster oder einem Drehknopf, vorzugsweise mittels eines mit der Hand betätigbaren Stellelements, stellbar. Des Weiteren kann / können wiederum eine durch den Anwender veränderbare oder nicht veränderbare Drehzahluntergrenze nₘᵢₙ und / oder eine Drehzahlobergrenze nₗₘ vorgesehen sein, die durch den Drehzahlgrenzwert n₀ nicht überschreitbar ist, wie dies in Verbindung mit der Figur 5 beschrieben ist.

Des Weiteren kann das in der Figur 6 dargestellte Steuer- und / oder Regelsetting vorzugsweise mit anderen Steuer- oder Regelcharakteristiken verbunden werden, insbesondere mit den in den Figuren 3 und 4 dargestellten Steuer- oder Regelcharakteristiken "A" - "G" oder beliebigen anderen Steuer- oder Regelcharakteristiken.

Eine Steuer- oder Regelvorrichtung ist mit einem Mikrokontroller vorgesehen, die / der zur Durchführung der Steuer- und / oder Regelcharakteristik der Figur 6 ausgebildet ist. Die Steuer- oder Regelvorrichtung ist mit den Stellelementen und zumindest einem Sensor, insbesondere einer Vorrichtung zur Bestimmung des Gasdrucks des Druckgases und / oder einem Drehmomentsensor und / oder einem Drehzahlsensor, operativ verbunden und ist des Weiteren insbesondere dazu ausgebildet, von dem zumindest einen Sensor Signale zu erhalten, mit Vorgabewerten (zum Beispiel einem vorgegebenen Gasdruck- oder Drehmomentwert, der Solldrehzahl n₁, Drehzahlobergrenze nₗₘ oder der Drehzahluntergrenze nₘᵢₙ) zu vergleichen und ein Signal zum Stellen des auf das Druckgas einwirkenden Ventils abzugeben.

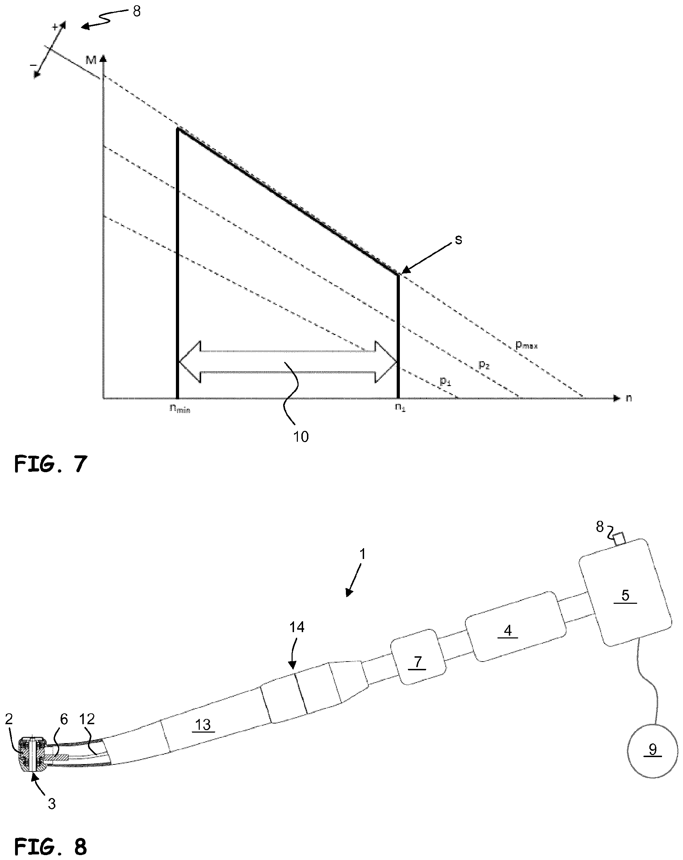

Das in der Figur 7 dargestellte Drehmoment-Drehzahl-Diagramm entspricht in den meisten Merkmalen dem Diagramm der Figur 5, so dass im Folgenden nur auf die Unterschiede eingegangen wird: Der wesentlichste Unterschied dieses Steuer- oder Regelsettings besteht darin, dass für den Anwender, insbesondere mit einem per Hand betätigbarem Stellelement 8, ein Gasdruckgrenzwert oder eine Obergrenze des Gasdrucks des Druckgases, welches das Laufrad antreibt, stellbar ist. Gemäß der Figur 7 ist der Gasdruckgrenzwert gleich dem maximalen, der Antriebsvorrichtung oder dem Laufrad zur Verfügung stehenden Gasdruck pₘₐₓ, durch Betätigen des Stellelements 8 ist der Gasdruckgrenzwert jedoch unter den maximalen Gasdruck pₘₐₓ absenkbar. Selbstverständlich ist es möglich, dass, wie in Verbindung mit der Figur 5 beschrieben, zusätzlich auch die als Solldrehzahl definierte Drehzahl n₁ durch ein Stellelement für den Anwender stellbar ist.

Ein weiterer Unterschied zur Figur 5 ist in der Position des Schaltpunktes S zu erkennen, die derart gewählt ist, dass im Wesentlichen erst bei Erreichen des maximalen Gasdrucks pₘₐₓ die Drehzahl reduziert wird.

Schließlich ist im Unterschied zu dem Ausführungsbeispiel der Figur 5 keine Drehzahlobergrenze nₗₘ vorgesehen.

Die Figur 8 zeigt eine medizinische, insbesondere dentale, Antriebsvorrichtung 1, die umfasst: Ein durch ein Druckgas in Bewegung versetzbares Laufrad 2, eine mit dem Laufrad 2 verbundene Werkzeughaltevorrichtung 3, eine Druckgasleitung 12 zur Versorgung des Laufrads 2 mit Druckgas, eine Drehzahlmessvorrichtung 6 zur Messung der Drehzahl des Laufrads 2 und / oder der Werkzeughaltevorrichtung 3, ein Ventil 4 zum Stellen des Gasdrucks und / oder des Durchflusses des Druckgases, vorzugsweise eine Vorrichtung 7 zur Bestimmung des Gasdrucks des Druckgases und eine Steuer- oder Regelvorrichtung 5, vorzugsweise mit einem Mikrokontroller. Die Steuer- oder Regelvorrichtung 5 ist operativ, zum Beispiel über einen Versorgungsschlauch oder drahtlos, mit der Drehzahlmessvorrichtung 6, mit der Vorrichtung 7 zur Bestimmung des Gasdrucks und mit dem Ventil 4 verbunden, so dass Drehzahlmessdaten und Gasdruckmessdaten an die Steuer- oder Regelvorrichtung 5 übertragbar und das Ventil 4 auf Basis der übertragenen Drehzahlmessdaten und / oder der Gasdruckmessdaten durch die Steuer- oder Regelvorrichtung 5 zum Stellen des Gasdrucks und / oder des Durchflusses des Druckgases betätigbar ist. Die Steuer- oder Regelvorrichtung 5 ist insbesondere zur Durchführung zumindest eines der im Vorstehenden beschriebenen Verfahren zur Steuerung und / oder Regelung einer mit Druckgas betreibbaren medizinischen, insbesondere dentalen, Antriebsvorrichtung und / oder zur Durchführung zumindest einer der im Vorstehenden beschriebenen Steuer- oder Regelcharakteristiken und / oder zur Durchführung zumindest einer der im Vorstehenden beschriebenen Steuer- oder Regelsettings ausgebildet.

Die Antriebsvorrichtung 1 umfasst vorzugsweise ein mit einer Hand greifbares Hand- oder Winkelstück 13, in dem zumindest das Laufrad 2, ein Teil der Druckgasleitung 12 und die Werkzeughaltevorrichtung 3 vorgesehen sind. Vorzugsweise ist das Hand- oder Winkelstück 13 über eine Kupplungsvorrichtung 14 lösbar mit der Steuer- oder Regelvorrichtung 5 verbunden. Mit der Steuer- oder Regelvorrichtung 5 verbunden oder daran vorgesehen sind des Weiteren das Stellelement oder die Bedienvorrichtung 8, welche(s) vom Anwender per Hand betätigbar ist, und das Stellelement oder die Bedienvorrichtung 9 (Fußsteuerung), welche(s) vom Anwender per Fuß betätigbar ist. Die Drehzahlmessvorrichtung 6 ist vorzugsweise im Hand- oder Winkelstück 13 vorgesehen und umfasst einen induktiven Drehzahlsensor mit einem am Laufrad 2 oder einem mit dem Laufrad 2 rotierenden Bauteil befestigten Magnetelement.

Bevorzugt ist an der Steuer- oder Regelvorrichtung eine Anzeige zur Anzeige eines für die Steuerung oder Regelung relevanten Parameters vorgesehen, zum Beispiel zumindest eines der im Vorstehenden genannten Drehzahl-, Drehmoment oder Gasdruckwerte.

Die Erfindung ist nicht auf die beschriebenen Ausführungsbeispiele beschränkt, sondern in den folgenden Patentansprüchen definiert.

## Patentansprüche

1. Von einem Mikrokontroller durchgeführtes Verfahren zur Steuerung oder Regelung einer mit Druckgas betreibbaren medizinischen, insbesondere dentalen, Antriebsvorrichtung (1) mit einem Laufrad (2), einer mit dem Laufrad (2) verbundenen Werkzeughaltevorrichtung (3) und einer Drehzahlmessvorrichtung (6) zur Messung der Drehzahl des Laufrads (2) oder der Werkzeughaltevorrichtung (3), wobei die Antriebsvorrichtung (1) von einer Druckgasquelle mit dem Druckgas mit einem maximalen Gasdruck versorgbar ist und wobei die Antriebsvorrichtung (1) ein Ventil (4) zum Stellen des Gasdrucks und des Durchflusses des Druckgases aufweist, wobei das Verfahren **dadurch gekennzeichnet ist, dass**
durch die Drehzahlmessvorrichtung (6) die Drehzahl des Laufrads (2) oder der Werkzeughaltevorrichtung (3) gemessen wird und bei Erreichen eines Drehzahlgrenzwertes (n₂, n₃) des Laufrads (2) durch Stellen des Ventils (4) der Gasdruck des Druckgases und die Drehzahl des Laufrads (2) verändert werden.

2. Von einem Mikrokontroller durchgeführtes Verfahren zur Steuerung oder Regelung nach Anspruch 1, **dadurch gekennzeichnet, dass**
bei Erreichen eines Drehzahlgrenzwertes (n₂, n₃) des Laufrads (2) und bei Erreichen eines Druckwertes (p₁) des Druckgases, der geringer ist als der maximale Gasdruck (pₘₐₓ) des Druckgases, durch Stellen des Ventils (4) der Gasdruck des Druckgases und die Drehzahl des Laufrads (2) verändert werden.

3. Von einem Mikrokontroller durchgeführtes Verfahren zur Steuerung oder Regelung nach Anspruch 1 oder 2, **dadurch gekennzeichnet, dass**
die Drehzahl des Laufrads (2), der Gasdruck des Druckgases und das Drehmoment des Laufrads (2) steigen.

4. Von einem Mikrokontroller durchgeführtes Verfahren zur Steuerung oder Regelung nach Anspruch 1 oder 2, **dadurch gekennzeichnet, dass**
bei einem bei Belastung eines in der Werkzeughaltevorrichtung (3) gehaltenen Werkzeugs der Antriebsvorrichtung (1) entgegenwirkenden, steigendend Drehmoment die Drehzahl des Laufrads (2) abnimmt und der Gasdruck des Druckgases steigt.

5. Von einem Mikrokontroller durchgeführtes Verfahren zur Steuerung oder Regelung nach einem der Ansprüche 1 - 4, **dadurch gekennzeichnet, dass**
der Drehzahlgrenzwert (n₂, n₃) und / oder der Druckwert (p₁) durch einen Anwender einstellbar ist / sind.

6. Von einem Mikrokontroller durchgeführtes Verfahren zur Steuerung oder Regelung nach einem der vorstehenden Ansprüche, **gekennzeichnet durch**
eine Drehzahluntergrenze (nₘᵢₙ), unterhalb der das Verfahren zur Steuerung und / oder Regelung nicht durchgeführt wird, wobei die Drehzahluntergrenze (nₘᵢₙ) > 0 s⁻¹ ist.

7. Von einem Mikrokontroller durchgeführtes Verfahren zur Steuerung oder Regelung nach einem der vorstehenden Ansprüche, **dadurch gekennzeichnet, dass**
wenn die Antriebsvorrichtung (1) nicht mit Druckgas versorgt wird, zumindest einer der folgenden Parameter durch den Anwender zumindest innerhalb eines vordefinierten Bereichs frei wählbar ist: Ein Drehzahlgrenzwert des Laufrads (2), der geringer ist als die durch den maximalen Gasdruck (pₘₐₓ) bestimmte maximale Drehzahl (nₘₐₓ), eine Solldrehzahl des Laufrads (2), ein Gasdruckgrenzwert des Druckgases, der geringer ist als der maximale Gasdruck (pₘₐₓ).

8. Steuer- oder Regelvorrichtung (5) für eine medizinische, insbesondere dentale, Antriebsvorrichtung (1), wobei die Antriebsvorrichtung (1) ein Laufrad (2), eine mit dem Laufrad (2) verbundene Werkzeughaltevorrichtung (3), eine Drehzahlmessvorrichtung (6) zur Messung der Drehzahl des Laufrads (2) oder der Werkzeughaltevorrichtung (3) und ein Ventil (4) zum Stellen des Gasdrucks und des Durchflusses des Druckgases aufweist, und wobei die Steuer- und / oder Regelvorrichtung (5) elektrisch betrieben ist und einen Mikrokontroller umfasst, **dadurch gekennzeichnet, dass**
die Steuer- oder Regelvorrichtung (5) zur Durchführung eines Verfahrens gemäß einem der vorstehenden Ansprüche ausgebildet ist, so dass durch die Drehzahlmessvorrichtung (6) die Drehzahl des Laufrads (2) oder der Werkzeughaltevorrichtung (3) gemessen wird und bei Erreichen eines Drehzahlgrenzwertes (n₂, n₃) des Laufrads (2) durch Stellen des Ventils (4) der Gasdruck des Druckgases und die Drehzahl des Laufrads (2) verändert werden.

9. Medizinische, insbesondere dentale, Antriebsvorrichtung (1), **gekennzeichnet durch** eine Steuer- oder Regelvorrichtung (5) nach Anspruch 8.

10. Medizinische, insbesondere dentale, Antriebsvorrichtung (1) nach Anspruch 9, **gekennzeichnet durch**
ein durch ein Druckgas in Bewegung versetzbares Laufrad (2), eine mit dem Laufrad (2) verbundene Werkzeughaltevorrichtung (3), eine Drehzahlmessvorrichtung (6) zur Messung der Drehzahl des Laufrads (2) oder der Werkzeughaltevorrichtung (3 und ein Ventil (4) zum Stellen des Gasdrucks und des Durchflusses des Druckgases, wobei die Steuer- oder Regelvorrichtung (5) operativ mit der Drehzahlmessvorrichtung (6) und mit dem Ventil (4) verbunden ist, so dass Drehzahlmessdaten an die Steuer- oder Regelvorrichtung (5) übertragbar sind und das Ventil (4) auf Basis der übertragenen Drehzahlmessdaten durch die Steuer- oder Regelvorrichtung (5) zum Stellen des Gasdrucks und des Durchflusses des Druckgases betätigbar ist.

11. Medizinische, insbesondere dentale, Antriebsvorrichtung (1) nach Anspruch 9, **gekennzeichnet durch**
ein durch ein Druckgas in Bewegung versetzbares Laufrad (2), eine mit dem Laufrad (2) verbundene Werkzeughaltevorrichtung (3), eine Drehzahlmessvorrichtung (6) zur Messung der Drehzahl des Laufrads (2) oder der Werkzeughaltevorrichtung (3), ein Ventil (4) zum Stellen des Gasdrucks und des Durchflusses des Druckgases und eine Vorrichtung (7) zur Bestimmung des Gasdrucks des Druckgases, wobei die Steuer- oder Regelvorrichtung (5) operativ mit der Drehzahlmessvorrichtung (6), mit der Vorrichtung (7) zur Bestimmung des Gasdrucks und mit dem Ventil (4) verbunden ist, so dass Drehzahlmessdaten und Gasdruckmessdaten an die Steuer- oder Regelvorrichtung (5) übertragbar sind und das Ventil (4) auf Basis der übertragenen Drehzahlmessdaten und / oder der Gasdruckmessdaten durch die Steuer- oder Regelvorrichtung (5) zum Stellen des Gasdrucks und des Durchflusses des Druckgases betätigbar ist.

12. Medizinische, insbesondere dentale, Antriebsvorrichtung (1) nach Anspruch 9, 10 oder 11, **gekennzeichnet durch**
zumindest ein von einem Anwender betätigbares Stellelement (8, 9) zum Festlegen zumindest eines der folgenden Parameter: Eines Drehzahlgrenzwerts des Laufrads (2), der geringer ist als die durch den maximalen Gasdruck pₘₐₓ bestimmte maximale Drehzahl (nₘₐₓ), einer Solldrehzahl des Laufrads (2), eines Gasdruckgrenzwerts des Druckgases, der geringer ist als der maximale Gasdruck (pₘₐₓ).

## Claims

1. A method performed by a microcontroller for controlling or regulating a compressed-gas-operable medical, in particular dental, drive device (1) comprising an impeller (2), a tool-holding device (3) connected to the impeller (2) and a rotational-speed-measuring device (6) for measuring the rotational speed of the impeller (2) or of the tool-holding device (3), wherein the drive device (1) can be supplied with the compressed gas at a maximum gas pressure from a compressed gas source and wherein the drive device (1) has a valve (4) for adjusting the gas pressure and the flow rate of the compressed gas, wherein the method is **characterized in that**
the rotational speed of the impeller (2) or of the tool-holding device (3) is measured by the rotational-speed-measuring device (6) and on reaching a rotational speed limit value (n₂, n₃) of the impeller (2) by adjusting the valve (4), the gas pressure of the compressed gas and the rotational speed of the impeller (2) are altered.

2. The method performed by a microcontroller for controlling or regulating according to Claim 1, **characterized in that**
on reaching a rotational speed limit value (n₂, n₃) of the impeller (2) and on reaching a pressure value (pi) of the compressed gas, which is lower than the maximum gas pressure (pₘₐₓ) of the compressed gas, the gas pressure of the compressed gas and the rotational speed of the rotor (2) are altered by adjusting the valve (4).

3. The method performed by a microcontroller for controlling or regulating according to claim 1 or 2, **characterized in that**
the rotational speed of the impeller (2), the gas pressure of the compressed gas and the torque of the rotor (2) increase.

4. The method performed by a microcontroller for controlling or regulating according to claim 1 or 2, **characterized in that**
the rotational speed of the rotor (2) decreases and the gas pressure of the compressed gas increases when the torque which counteracts the drive device (1) increases when a load acts on a tool held in the tool-holding device (3).

5. The method performed by a microcontroller for controlling or regulating according to any one of claims 1 - 4, **characterized in that**
the rotational speed limit value (n₂, n₃) and/or the pressure value (p₁) is/are adjustable by a user.

6. The method performed by a microcontroller for controlling or regulating according to any one of the preceding claims, **characterized in that**
a rotational speed lower limit (nₘᵢₙ) below which the method for controlling and/or regulating is not performed, wherein the rotational speed lower limit (nₘᵢₙ) is > 0 s⁻¹.

7. The method performed by a microcontroller for controlling or regulating according to any one of the preceding claims, **characterized in that**
when the drive device (1) is not supplied with compressed gas, at least one of the following parameters is freely selectable by the user at least within a predefined range: a rotational speed limit value of the impeller (2) which is lower than the maximum rotational speed (nₘₐₓ) defined by the maximum gas pressure (pₘₐₓ), a setpoint rotational speed of the impeller (2), a gas pressure limit value of the compressed gas which is lower than the maximum gas pressure (pₘₐₓ).

8. A controlling or regulating device (5) for a compressed-gas-operable medical, in particular dental, drive device (1), wherein the drive device (1) comprises an impeller (2), a tool-holding device (3) connected to the impeller (2), a rotational-speed-measuring device (6) for measuring the rotational speed of the impeller (2) or of the tool-holding device (3), and a valve (4) for adjusting the gas pressure and the flow rate of the compressed gas, and wherein the controlling and/or regulating device (5) is operated electrically and comprises a microcontroller, **characterized in that**
the control or regulating device (5) is configured to perform a method according to any one of the preceding claims, so that the rotational speed of the impeller (2) or of the tool-holding device (3) is measured by the rotational-speed-measuring device (6) and on reaching a rotational speed limit value (n₂, n₃) of the impeller (2) by adjusting the valve (4), the gas pressure of the compressed gas and the rotational speed of the impeller (2) are altered.

9. A medical, in particular dental, drive device (1), **characterized by**
a controlling or regulating device (5) according to Claim 8.

10. The medical, in particular dental, drive device (1) according to Claim 9, **characterized by**
an impeller (2) which can be set in motion by a compressed gas, a tool-holding device (3) which is connected to the impeller (2), a rotational-speed-measuring device (6) for measuring the rotational speed of the impeller (2) or of the tool-holding device (3), and a valve (4) for adjusting the gas pressure and the flow rate of the compressed gas, wherein the control or regulating device (5) is operatively connected to the rotational-speed-measuring device (6) and to the valve (4), so that measured data on the rotational speed can be transmitted to the control or regulating device (5) and the valve (4) can be operated on the basis of the transmitted measured data on the rotational speed by the control or regulating device (5) for adjusting the gas pressure and the flow rate of the compressed gas.

11. The medical, in particular dental, drive device (1) according to Claim 9, **characterized by**
an impeller (2) which can be set in motion by a compressed gas, a tool-holding device (3) which is connected to the impeller (2), a rotational-speed-measuring device (6) for measuring the rotational speed of the impeller (2) or of the tool-holding device (3), a valve (4) for adjusting the gas pressure and the flow rate of the compressed gas and a device (7) for determining the gas pressure of the compressed gas, wherein the control or regulating device (5) is operatively connected to the rotational-speed-measuring device (6), to the device (7) for determining the gas pressure and to the valve (4), so that measured data on the rotational speed and measured data on the gas pressure can be transmitted to the control or regulating device (5) and the valve (4) can be operated on the basis of the transmitted measured data on the rotational speed and/or the measured data on the gas pressure by the control or regulating device (5) for adjusting the gas pressure and the flow rate of the compressed gas.

12. The medical, in particular dental, drive device (1) according to Claim 9, 10 or 11, **characterized by**
at least one actuator element (8, 9) operable by a user for setting at least one of the following parameters: a rotational speed limit value of the impeller (2) which is lower than the maximum rotational speed (nₘₐₓ) determined by the maximum gas pressure (pₘₐₓ), a setpoint rotational speed of the impeller (2), a gas pressure limit value of the compressed gas which is lower than the maximum gas pressure (pₘₐₓ).

## Revendications

1. Procédé mis en oeuvre par un microcontrôleur pour la commande ou régulation d'un dispositif d'entraînement médical (1), en particulier dentaire, pouvant être exploité avec un gaz sous pression, avec un rotor (2), un dispositif porte-outil (3) relié au rotor (2) et un dispositif de mesure de vitesse de rotation (6) pour mesurer la vitesse de rotation du rotor (2) ou du dispositif porte-outil (3), dans lequel le dispositif d'entraînement (1) peut être alimenté par une source de gaz sous pression avec le gaz sous pression avec une pression de gaz maximale et dans lequel le dispositif d'entraînement (1) présente une soupape (4) pour régler la pression de gaz et le débit du gaz sous pression, dans lequel le procédé est **caractérisé en ce que**,
la vitesse de rotation du rotor (2) ou du dispositif porte-outil (3) est mesurée par le dispositif de mesure de vitesse de rotation (6) et à l'atteinte d'une valeur limite de vitesse de rotation (n₂, n₃) du rotor (2), par réglage de la soupape (4), la pression de gaz du gaz sous pression et la vitesse de rotation du rotor (2) sont modifiés.

2. Procédé mis en oeuvre par un microcontrôleur pour la commande ou régulation selon la revendication 1, **caractérisé en ce que**
à l'atteinte d'une valeur limite de vitesse de rotation (n₂, n₃) du rotor (2) et à l'atteinte d'une valeur de pression (p₁) du gaz sous pression, laquelle est inférieure à la pression de gaz maximale (pₘₐₓ) du gaz sous pression, la pression de gaz du gaz sous pression et la vitesse de rotation du rotor (2) sont modifiées par réglage de la soupape (4).

3. Procédé mis en oeuvre par un microcontrôleur pour la commande ou régulation selon la revendication 1 ou 2, **caractérisé en ce que**
la vitesse de rotation du rotor (2), la pression de gaz du gaz sous pression, et le couple du rotor (2) augmentent.

4. Procédé mis en oeuvre par un microcontrôleur pour la commande ou régulation selon la revendication 1 ou 2, **caractérisé en ce que**
en cas d'un couple augmentant, agissant à l'encontre du dispositif d'entraînement (1), en cas de sollicitation d'un outil maintenu dans le dispositif porte-outil (3), la vitesse de rotation du rotor (2) diminue et la pression de gaz du gaz sous pression augmente.

5. Procédé mis en oeuvre par un microcontrôleur pour la commande ou régulation selon l'une des revendications 1-4, **caractérisé en ce que**
la valeur limite de vitesse de rotation (n₂, n₃) et/ou la valeur de pression (p₁) est/sont réglable(s) par un utilisateur.

6. Procédé mis en oeuvre par un microcontrôleur pour la commande ou régulation selon l'une des revendications précédentes, **caractérisé par**
une limite inférieure de vitesse de rotation (nₘᵢₙ) en-deçà de laquelle le procédé pour la commande et/ou régulation n'est pas mis en oeuvre, dans lequel la limite inférieure de vitesse de rotation est de (nₘᵢₙ) > 0 s⁻¹.

7. Procédé mis en oeuvre par un microcontrôleur pour la commande ou régulation selon l'une des revendications précédentes, **caractérisé en ce que**,
lorsque le dispositif d'entraînement (1) n'est pas alimenté avec du gaz sous pression, au moins l'un des paramètres suivants peut être librement sélectionné par l'utilisateur au moins dans les limites d'une plage prédéfinie: une valeur limite de vitesse de rotation du rotor (2), laquelle est inférieure à la vitesse de rotation maximale (nₘₐₓ) déterminée par la pression de gaz maximale (pₘₐₓ), une vitesse de rotation de consigne du rotor (2), une valeur limite de pression de gaz du gaz sous pression, laquelle est inférieure à la pression de gaz maximale (pₘₐₓ).

8. Dispositif de commande ou de régulation (5) pour un dispositif d'entraînement médical (1), en particulier dentaire, dans lequel le dispositif d'entraînement (1) présente un rotor (2), un dispositif porte-outil (3) relié au rotor (2), un dispositif de mesure de vitesse de rotation (6) pour mesurer la vitesse de rotation du rotor (2) ou du dispositif porte-outil (3), et une soupape (4) pour régler la pression de gaz et le débit du gaz sous pression, et dans lequel le dispositif de commande ou de régulation (5) est exploité électriquement et comprend un microcontrôleur, **caractérisé en ce que**
le dispositif de commande ou de régulation (5) est réalisé pour la mise en oeuvre d'un procédé selon l'une des revendications précédentes de sorte que grâce au dispositif de mesure de vitesse de rotation (6), la vitesse de rotation du rotor (2) ou du dispositif porte-outil (3) est mesurée et qu'à l'atteinte d'une valeur limite de vitesse de rotation (n₂, n₃) du rotor (2), par réglage de la soupape (4), la pression de gaz du gaz sous pression et la vitesse de rotation du rotor (2) sont modifiées.

9. Dispositif d'entraînement médical (1), en particulier dentaire, **caractérisé par** un dispositif de commande ou de régulation (5) selon la revendication 8.

10. Dispositif d'entraînement médical (1), en particulier dentaire selon la revendication 9, **caractérisé par**
un rotor (2) pouvant être mis en mouvement par un gaz sous pression, un dispositif porte-outil (3) relié au rotor (2), un dispositif de mesure de vitesse de rotation (6) pour la mesure de la vitesse de rotation du rotor (2) ou du dispositif porte-outil (3), et une soupape (4) pour régler la pression de gaz et le débit du gaz sous pression, dans lequel le dispositif de commande ou de régulation (5) est relié de manière opérative au dispositif de mesure de vitesse de rotation (6) et à la soupape (4) de sorte que des données de mesure de vitesse de rotation peuvent être transmises au dispositif de commande ou de régulation (5) et que la soupape (4) peut être actionnée sur la base des données de mesure de vitesse de rotation transmises par le dispositif de commande ou de régulation (5) pour régler la pression de gaz et le débit du gaz sous pression.

11. Dispositif d'entraînement médical (1), en particulier dentaire selon la revendication 9, **caractérisé par**
un rotor (2) pouvant être mis en rotation par un gaz sous pression, un dispositif porte-outil (3) relié au rotor (2), un dispositif de mesure de vitesse de rotation (6) pour la mesure de la vitesse de rotation du rotor (2) ou du dispositif porte-outil (3), une soupape (4) pour régler la pression de gaz et le débit du gaz sous pression, et un dispositif (7) pour déterminer la pression de gaz du gaz sous pression, dans lequel le dispositif de commande ou de régulation (5) est relié de manière opérative au dispositif de mesure de vitesse de rotation (6), au dispositif (7) pour déterminer la pression de gaz et à la soupape (4) de sorte que des données de mesure de vitesse de rotation et des données de mesure de pression de gaz peuvent être transmises au dispositif de commande ou de régulation (5) et que la soupape (4) peut être actionnée sur la base des données de mesure de vitesse de rotation et/ou des données de mesure de pression de gaz transmises par le dispositif de commande ou de régulation (5) pour régler la pression de gaz et le débit du gaz sous pression.

12. Dispositif d'entraînement médical (1), en particulier dentaire selon la revendication 9, 10 ou 11, **caractérisé par**
au moins un élément de réglage (8, 9) actionnable par un utilisateur pour déterminer au moins l'un des paramètres suivants: une valeur limite de vitesse de rotation du rotor (2), laquelle est inférieure à la vitesse de rotation maximale (nₘₐₓ) déterminée par la pression de gaz maximale pₘₐₓ, une vitesse de rotation de consigne du rotor (2), une valeur limite de pression de gaz du gaz sous pression, laquelle est inférieure à la pression de gaz maximale (pₘₐₓ).
